# EUROPEAN PATENT SPECIFICATION

(11) **EP 4 129 076 B1**
(45) Date of publication and mention of the grant of the patent: **11.02.2026**
(21) Application number: 21775205.4
(22) Date of filing: 26.03.2021
(51) Int. Cl.: A23J 3/00, A23J 3/16, A23C 20/02, A23C 20/00, C12N 9/22, C12R 1/46

(54) **METHOD FOR MANUFACTURING PLANT-BASED CHEESE-LIKE FOOD**
VERFAHREN ZUR HERSTELLUNG EINES KÄSEÄHNLICHEN NAHRUNGSMITTELS AUF PFLANZLICHER BASIS
PROCÉDÉ DE FABRICATION D'ALIMENT DE TYPE FROMAGE À BASE DE PLANTES

(30) Priority: 26.03.2020 JP 2020055406
(43) Date of publication of application: 08.02.2023
(73) Proprietor: Fuji Oil Co., Ltd., Izumisano-shi, Osaka 598-8540 (JP)
(72) Inventor: YANAGISAWA Masanobu, Izumisano-shi, Osaka 598-8540 (JP)
(74) Representative: Becker, Eberhard
(86) International application number: PCT/JP2021/012767
(87) International publication number: WO 2021/193892

(56) References cited:
- EP-B1- 2 943 077
- WO-A1-2006/135089
- CN-A- 102 144 673
- JP-A- 2006 296 422
- JP-A- H03 224 448
- US-A- 3 857 970
- HELLER KNUT J ET AL: "Behaviour of Genetically Modified Microorganisms In Yoghurt", SYSTEMATIC AND APPLIED MICROBIOLOGY, vol. 18, no. 4, 1 December 1995 (1995-12-01), AMSTERDAM, NL, pages 504 - 509, XP093075929, ISSN: 0723-2020, DOI: 10.1016/S0723-2020(11)80410-8
- HELLER K J ET AL: "VERHALTEN GENETISCH MODIFIZIERTER MIKROORGANISMEN IN JOGHURT. �(BEHAVIOUR OF GENETICALLY MODIFIED MICROORGANISMS IN YOGHURT)", MILCHWISSENSCHAFT, VV GMBH VOLKSWIRTSCHAFTLICHER VERLAG. MUNCHEN, DE, vol. 51, no. 11, 1 January 1996 (1996-01-01), pages 653, XP000637654, ISSN: 0026-3788
- HOLS P ET AL: "New insights in the molecular biology and physiology of Streptococcus thermophilus revealed by comparative genomics", FEMS MICROBIOLOGY REVIEWS, ELSEVIER, AMSTERDAM; NL, vol. 29, no. 3, 1 August 2005 (2005-08-01), pages 435 - 463, XP027666195, ISSN: 0168-6445, [retrieved on 20050801]
- "Applied Mycology and Biotechnology - Volume 5, Genes and Genomics", 1 January 2005, ELSEVIER B. V., ISBN: 978-0-444-51808-8, article P�GGELER STEFANIE: "Fungal Intervening Sequences - Chapter 3", pages: 71 - 92, XP093076162, DOI: 10.1016/S1874-5334(05)80005-X
- MASACHIKA IRIE: "Isolation and Properties of a Ribonuclease from Aspergillus saitoi", JOURNAL OF BIOCHEMISTRY, vol. 62, no. 5, 1 November 1967 (1967-11-01), GB, pages 509 - 518, XP093076226, ISSN: 0021-924X, DOI: 10.1093/oxfordjournals.jbchem.a128700

## Description

### Technical Field

The present invention relates to a method for producing a plant-based cheese-like food product.

### Background Art

Cheese is a type of dairy product produced from milk collected from cows, water buffaloes, sheep, goats, yaks, or the like through processes, such as coagulation and fermentation. Cheese is not only consumed as it is, but also used in combination with other food products. For example, it is used in various cuisines, such as pizza, pasta, gratin, risotto, and cheese fondue, and is growing in demand every year.

On the other hand, the rapid increase in consumption of animal food materials in recent years has also consequently brought serious health problems, such as obesity and diabetes, and there is a growing interest in improving health through dietary habits. In addition, from the viewpoint of environmental awareness and animal welfare, millennials and later generations tend to consciously consume non-animal food products. Under such circumstances, plant protein food products are highly appreciated.

The above circumstances have led to an increase in demand for food products made from soybeans, peas, or the like as raw materials, and efforts have been made to incorporate plant proteins into a wide range of food products.

For example, some studies have been conducted for possible use of plant protein food products to substitute common dairy products, such as cheese and yogurt. And soybean materials, such as soymilk, isolated soy proteins, and soy flour, are used as raw materials containing a planted-based protein (Patent Literatures 1 to 3). Patent Literature 4 relates to a process for making a cheese-like foodstuff having good texture and body characteristics from soy protein, without adding milk solids, alkaline metal caseinates, or other texture reinforcement additives, wherein proteolytic enzymes can be added during the process in order to minimize the be any taste of soy milk.

### Citation List

### Patent Literature

Patent Literature 1: JP 2006-135089 A
Patent Literature 2: JP 2014-233270 A
Patent Literature 3: JP 2015-008691 A
Patent Literature 4: US 3857970 A

### Summary of Invention

### Technical Problem

However, plant-based cheese-like food products generally tend to have less umami compared with cheese made from milk. A method of adding umami seasoning is available, but imparting cheese-like natural umami is difficult, and use of chemical seasoning may not be accepted because of the growing awareness toward natural origin products.

In response to the above circumstances, an object of the present invention is to provide a method for producing a plant-based cheese-like food product with enhanced cheese-like umami similar to that of cheese made from milk.

### Solution to Problem

As a result of diligent research to solve the above problems, the present inventors found that the above problems can be solved by treating plant-based milk, such as soymilk, with a nuclease in advance and then acidifying the plant-based milk, and completed the present invention.

That is, the present invention includes the following technical ideas:
A method for producing a plant-based cheese-like food product, the method comprising applying a nuclease to a raw material containing a plant protein, wherein the nuclease is 5'-phosphodiesterase, and then acidifying the raw material to a pH of 3.5 to 5.7, and wherein animal-derived raw material accounts for less than 50 wt.% in all raw materials of the plant-based cheese-like food product.

In one embodiment of the method for producing a plant-based cheese-like food product according to the present invention, the acidification is lactic acid fermentation, wherein optionally the raw material containing a plant protein is derived from a legume, a seed or a cereal.

In another embodiment of the method for producing a plant-based cheese-like food product according to the present invention, wherein the acidification is lactic acid fermentation, an oil or fat is optionally added as a raw material. In addition to said oil or fat, a thickener is also optionally added as a raw material.

In another embodiment of the method for producing a plant-based cheese-like food product according to the present invention, the nuclease performs hydrolysis at a temperature of 35°C to 75°C.

### Advantageous Effects of Invention

The present invention is defined in the appended claims and can provide a plant-based cheese-like food product with enhanced cheese-like umami similar to that of cheese made from milk.

### Description of Embodiments

A method for producing a plant-based cheese-like food product of the present invention is defined by the appended claims and is characterized by including applying a nuclease to a raw material containing a plant protein, and then acidifying the raw material. The present invention will be described in detail below.

### (Plant-Based Cheese-Like Food)

The plant-based cheese-like food product obtained by the method of the present invention is a food product having a cheese-like product form, flavor, and texture and produced based on a plant protein and optionally a plant raw material, such as a plant-based oil or fat, and starch, as necessary, and is also used as a substitute food product of cheese. The product form can be of any cheese-like product, such as a block, shred, powder, or paste form. Here, the term "plant-based" does not mean that an animal raw material is not contained at all, and specifically means that the animal-derived raw material accounts for less than 50 wt.% in all raw materials. In a more preferred aspect, the animal-derived raw material accounts for 40 wt.% or less, 30 wt.% or less, 20 wt.% or less, 10 wt.% or less, or 0 wt.% in all raw materials.

### (Plant Protein)

The plant-based cheese-like food product obtained by the method of the present invention contains at least a plant protein as a protein. For the raw material containing the plant protein, a powdery protein, such as a concentrated protein or an isolated protein; a plant-based milk; a legume flour; or the like can be used. The type of plant is not limited, but examples include legumes represented by soybeans, peas, and fava beans; seeds represented by almond, hazelnuts, cashew nuts, walnuts, peanuts, and pistachios; and cereals represented by rice and oats. In addition, these can be mixed and used in appropriate proportions.

In a certain aspect, the plant protein content in the plant-based cheese-like food product is appropriately from 0.1 to 15 wt.% from the viewpoint of viscosity during the food product preparation, and the content is preferably from 0.5 to 10 wt.% and more preferably from 1 to 3 wt.%.

When plant-based milk, such as soymilk, is used, for example, a typical soymilk can be used which is obtained through water extraction from soybeans, such as whole soybeans or defatted soybeans, and removal of soy pulp as insoluble fibers. At that time, to obtain soymilk with a better flavor, an improved production method can be appropriately applied. In addition, the soymilk in a slurry form can also be used in which soy pulp is finely ground rather than being removed from the soymilk.

In one suitable aspect, when a low-fat soy protein raw material with a fat content as low as that of skimmed milk powder is desired to be used, the use of a low-fat or non-fat soymilk is desirable than the use of defatted soymilk obtained from defatted soybeans in that the flavor can be adjusted to have more strong umami with less unpleasant taste. Here, the low-fat or non-fat soymilk is obtained by producing soymilk or the like from whole soybeans and additionally centrifuging the produced soymilk or the like to remove a cream layer rich in a low specific gravity oil. The use of such low-fat or non-fat soymilk is more preferred than the use of defatted soymilk obtained from defatted soybeans in that the flavor can be adjusted to have more strong umami with less unpleasant taste. In this case, a lipid content of the low-fat or non-fat soymilk is preferably 5 wt.% or less in dry matter and more preferably 2 wt.% or less in dry matter. In addition, a protein/carbohydrate ratio by weight of the low-fat or non-fat soymilk is preferably from 0.01 to 2, more preferably from 0.5 to 2, and even more preferably from 1 to 2 in that the flavor can be adjusted to have more strong umami. In a more suitable aspect, for the composition of soy proteins, "lipophilic proteins" among soy proteins are preferably further reduced from the viewpoint of a flavor with less unpleasant taste. For such a soy protein raw material, a fat-reduced soy protein material described in JP 2012-16348 A can be used. An index of whether the lipophilic proteins are reduced can be estimated by determining a lipophilic proteins content index (LCI) value described in JP 2012-16348 A above. In an embodiment of the present invention, a fat-reduced soy protein material with an LCI value of 40% or less, in which lipophilic proteins are reduced, is preferably used.

In addition, in another suitable aspect, when a soy protein raw material with a rich flavor like a milk-derived fresh cream is desired to be used, a "soymilk cream", a soybean emulsion composition rich in lipid and protein, is also preferably used in that the raw material can be adjusted to have a rich flavor. For the soymilk cream, for example, a cream obtained by collecting a cream layer rich in low specific gravity oil can be used, the cream layer being produced by additional centrifugation of soymilk obtained from whole soybeans. The lipid content of the soymilk cream is preferably 35 wt.% or greater and more preferably from 40 to 75 wt.% in dry matter. In addition, the protein content of the soymilk cream is preferably 25 wt.% or greater and more preferably 30 wt.% or greater in dry matter. In this case, the lipid/protein ratio by weight of the soymilk cream is preferably 1 or greater, more preferably 1.5 or greater, and even more preferably 2 or greater.

In a more suitable aspect of the soymilk cream, "lipophilic proteins" among soy proteins are preferably concentrated from the viewpoint that it has a richer body and a soy-derived good flavor. For such a soy protein raw material, a soybean emulsion composition described in JP 2012-16348 A can be used. An index of whether the lipophilic proteins are concentrated can be estimated by determining a lipophilic proteins content index (LCI) value described in JP 2012-16348 A above. In an embodiment of the present invention, a soymilk cream with an LCI value of 55% or greater, in which lipophilic proteins are concentrated, is preferably used.

### (Nuclease)

In the method for producing a plant-based cheese-like food product of an embodiment of the present disclosure, which does not form part of the present invention, application of a nuclease to a raw material containing a plant protein is important. The nuclease may be any type of enzyme involved in the hydrolysis of a nucleic acid or its degradation product, a nucleotide or a nucleoside, and specific examples include nucleases, such as ribonucleases and deoxyribonucleases. Among these, a ribonuclease, an enzyme involved in ribonucleic acid (RNA) degradation, is preferred because most of the nucleic acids in living organisms are RNAs. For the ribonuclease, an endonuclease or an exonuclease can be used, and in a more preferred aspect, examples include 5'-phosphodiesterase, which is an exonuclease. In the method for producing a plant-based cheese-like food product according to the present invention, the used nuclease is 5'-phosphodiesterase.

In addition, the unit of enzyme activity, for example, 1 unit for 5'-phosphodiesterase activity, is expressed as an amount of enzyme that releases 1 µmol of phosphoric acid per minute when the enzyme is applied to a substrate, which is adenosine-3'-monophosphate (3'-AMP), under conditions of pH 5.0 and 70°C.

Furthermore, contamination of the nuclease with a protease if any may cause bitterness due to degradation of the protein, and thus the protease activity is preferably low. The protease activity of 50000 PUN or less is appropriate, and it may be preferably 30000 PUN or less and more preferably 10000 PUN or less. PUN represents an activity expressed by Folin color development corresponding to 1 µg of tyrosine released per minute as a TCA-soluble component when 1 mL of an enzyme solution is added to 5 mL of 0.6% milk casein (pH 7.5, M/25 phosphate buffer) and reacted at 30°C for 10 minutes.

The treatment conditions of hydrolysis by a nuclease are not particularly limited, but the hydrolysis is preferably performed at an optimum temperature and an optimum pH of the nuclease, specifically, at 20 to 80°C, preferably at 30 to 80°C, and more preferably at 35 to 75°C.

After the hydrolysis, a nuclease inactivation process may be added as necessary. The inactivation conditions are not particularly limited as long as the temperature is not lower than the temperature at which the nuclease is inactivated.

### (Acidification)

The plant-based cheese-like food product obtained by the method of the present invention is obtained by acidifying the raw material containing a plant protein to which the nuclease described above has been applied. In the method of the present invention, the acidification of the raw material is to a pH of 3.5 to 5.7, and preferably pH 4 to 5.5. The acidification to an excessively high pH would tend to impair the shelf life. The acidification to an excessively low pH would give a strong sour taste and, when used as a soy protein-containing cheese-like food product, the balance of the food product as a whole may be compromised. And thus, the pH is appropriately adjusted to be within the above range.

The acidification can be performed using an organic acid in a certain aspect. Examples of the organic acid that can be used include citric acid, lactic acid, malic acid, fumaric acid, gluconic acid, and phytic acid.

The acidification can be performed by lactic acid fermentation in a certain aspect. In this case, the lactic acid fermentation is performed at 15 to 45°C using a lactic acid bacteria starter until reaching the above pH range. In addition, the lactic acid fermentation may be stopped some time before completion, and then the pH may be finally adjusted to the above pH range with an alkali, an organic acid, an inorganic acid, or the like.

### (Additional Raw Material)

In the method for producing a plant-based cheese-like food product according to an embodiment of the present invention, an additional raw material besides the plant protein can be added according to a desired quality of the product, or an additional raw material does not need to be added.

### Oil or Fat

For the oil or fat, those that is edible and has a melting point of approximately 20 to 50°C can be widely adopted and can be exemplified by plant-based oils and fats, such as rapeseed oil, soybean oil, sunflower oil, cottonseed oil, peanut oil, rice bran oil, corn oil, safflower oil, olive oil, kapok oil, sesame oil, evening primrose oil, palm oil, shea butter, sal fat, cacao butter, coconut oil, and palm kernel oil; and animal oils and fats, such as milk fat, beef tallow, pork fat, fish oil, and whale oil. Examples can include single oils or fats or mixed oils of the above oils or fats, or processed oils or fats obtained by curing, separation, transesterification, or the like of those.

The plant-based cheese-like food product obtained by the method of the present invention may contain from 10 to 50 wt.%, preferably from 15 to 45 wt.%, and even more preferably from 20 to 40 wt.% of an oil or fat when the oil or fat is added.

### Thickener

The plant-based cheese-like food product obtained by the method of the **present** invention preferably further contains a thickener because this can impart more viscosity to the plant-based cheese-like food product and further improves process suitability for a shaping process, such as shredding. For the thickener, a processed starch, such as hydroxypropylated starch; locust bean gum; or guar gum are preferably used.

### Emulsifier

In the method for producing a plant-based cheese-like food product according to an embodiment of the present invention, an emulsifier can be used to an extent that does not interfere with the flavor. The emulsifier is not particularly limited, and an emulsifier known in the art can be used and can be exemplified by lecithin, sucrose fatty acid esters, propylene glycol fatty acid esters, sorbitan fatty acid esters, glycerin fatty acid esters, polyglycerin fatty acid esters; various organic acid monoglycerides, such as acetic acid monoglyceride, tartaric acid monoglyceride, acetic acid-tartaric acid mixed monoglyceride, citric acid monoglyceride, diacetyl tartaric acid monoglyceride, and lactic acid monoglyceride; and polyoxyethylene sorbitan fatty acid esters.

In addition to the above, for the method for producing a plant-based cheese-like food product according to an embodiment of the present invention, a flavor, such as a milky flavor, a cheese flavor, or a soymilk flavor; spices of various types; or a fruit puree or a jam can be used to impart a flavor. A sweetener, such as sucralose, aspartame, or stevia, can be used to impart sweetness, and a colorant, such as beta-carotene, a paprika pigment, or an annatto dye, can be used for coloring. In addition, a shelf life improver, such as glycine, sodium acetate, or egg white lysozyme, can also be used to improve the shelf life.

### (Production Aspect)

In one aspect of method of producing the soy protein-containing cheese-like food product an oil-in-water emulsion in which a raw material containing an oil or fat and a plant protein, a starch, a salt, a pigment, water, and the like are mixed is pre-emulsified and homogenized, then subjected to sterilization and cooling processes, and the soy protein-containing cheese-like food product can be produced. In addition, the acidification is performed during the pre-emulsification. The heat sterilization is performed also to gelatinize the starch and is preferably performed at 70 to 95°C.

### Examples

Examples will be shown below, and details of the present invention will be more specifically described. In the examples, values in "part" or "%" are all expressed by weight.

### Test Example 1 (not according to the present invention)

The following were prepared as raw materials containing a plant protein.
- Low-fat soymilk (available from Fuji Oil Co., Ltd., solid content: 90.2%, protein: 5.1%, lipid: 0.5%, and protein/carbohydrate ratio: 1.6)
- Soymilk cream (available from Fuji Oil Co., Ltd., solid content: 81.0%, protein: 5.6%, and lipid: 12.3%)
- Soy flour solution (solid content: 8.8%, protein: 3.9%, and lipid: 1.9%)
- Almond milk (available from Tsukuba Dairy Products Co., Ltd., total solids content 9.9%, protein content 2.9%, and lipid content 5.6%)
- Oat drink (The Bridge, protein content 0.7% and lipid content 1.9%)

To each raw material described above, 0.1% of a nuclease "Amano G" (5'-phosphodiesterase, available from Amano Enzyme Inc.) was added, and enzymatic treatment was performed under reaction conditions of 70°C for 2 hours. After the enzymatic treatment, sterilization was performed at 144°C for 4 seconds in a direct steam heat sterilizer, the treated solution was cooled, and an enzyme-treated solution was obtained.

A sensory evaluation was performed by comparing the flavor of each of the resulting enzyme-treated solutions with the flavor of each raw material before the reaction. Five panelists belonging to the cheese development department and skilled in cheese flavor evaluation were asked to perform the sensory evaluation by assigning a score of 1 to 5 to each sample by consensus based on the evaluation criteria below. "Umami" was evaluated also in consideration of whether the umami had a suitable quality as the flavor of cheese. Whether the umami had a suitable quality as the flavor of cheese was evaluated specifically based on the flavor of cheese made from milk, and each panelist determined qualitatively whether the umami matched glutamic acid- or other components-based umami present in the flavor of cheese made from milk. The results are shown in Table 1.

### <Evaluation Criteria>

1: Umami is not improved at all compared with that before the reaction
2: Umami is improved little compared with that before the reaction
3: Umami is improved compared with that before the reaction
4: Umami is fairly improved compared with that before the reaction
5: Umami is significantly improved compared with that before the reaction

**Table 1**

| | Sensory evaluation score |
|---|---|
| Low-fat soymilk | 5 |
| Soymilk cream | 3 |
| Soy flour solution | 4 |
| Almond milk | 5 |
| Oat drink | 4 |

As shown in Table 1, an effect of improving the umami was found in all the raw materials compared with those before the reaction, though with variations.

### Test Example 2

To 80 parts of each of the raw materials after the enzymatic treatment obtained in Test Example 1 and the raw materials before the enzymatic treatment, 1 part of glucose and 19.99 parts of water were added, then 0.01 parts of lactic acid bacterium (*Streptococcus thermophilus*) was added, and lactic acid fermentation was performed at 40°C until pH 4.6 was reached, and a flavor evaluation was performed in the same manner as in Test Example 1. The results are shown in Table 2.

**Table 2**

| | Sensory evaluation score |
|---|---|
| Low-fat soymilk | 5 |
| Soymilk cream | 4 |
| Soy flour solution | 5 |
| Almond milk | 5 |
| Oat drink | 5 |

As shown in Table 2, the enzymatic treatment combined with the lactic acid fermentation tended to further enhance the umami.

### Example 1

A plant-based cheese-like food product was produced as follows using the enzyme-treated and lactic acid-fermented soymilk cream prepared in Test Example 2.

That is, 36 parts of a palm mid fraction in palm oil (slip melting point 30°C/SFC: 90% at 10°C and 80% at 20°C), 30 parts of the enzyme-treated and lactic acid-fermented soymilk cream obtained in Test Example 2, 11 parts of a processed starch derived from tapioca, 1 part of salt, 22 parts of water, 1.1 parts of a pH adjuster containing lactic acid, and 0.002 parts of a pigment were mixed at 55°C for 10 minutes to pre-emulsify, and homogenized under a pressure of 100 kg/cm². After homogenization, the mixture was passed through a scraping continuous heat exchanger, sterilized by heating at 80 to 90°C, filled, rapidly cooled in a tunnel freezer, then aged in a refrigerator, and a soy protein-containing cheese-like food product was obtained. In addition, a cheese-like food product was obtained as a comparison (comparative example) in the same manner except using a lactic acid-fermented soymilk cream without the enzymatic treatment of Test Example 2.

A flavor evaluation was performed in the same manner as in Test Example 1: the addition of the enzymatic treatment tended to enhance the umami in the same manner as in the simple evaluation system of Test Example 2. In addition, the umami had a suitable quality as the flavor of cheese.

### Examples 2 to 5

Samples of plant-based cheese-like food products were prepared in the same manner as in Example 1 also for the enzyme-treated and lactic acid-fermented low-fat soymilk, soy flour solution, almond milk, and oat drink obtained in Test Example 2. A flavor evaluation was performed in the same manner as in Test Example 1. The samples having undergone the enzymatic treatment tended to have enhanced umami over the samples subjected to the lactic acid fermentation only, and the umami had a suitable quality as the flavor of cheese.

## Claims

1. A method for producing a plant-based cheese-like food product, the method comprising applying a nuclease to a raw material containing a plant protein, wherein the nuclease is 5'-phosphodiesterase, and then acidifying the raw material to a pH of 3.5 to 5.7, and wherein animal-derived raw material accounts for less than 50 wt.% in all raw materials of the plant-based cheese-like food product.

2. The method for producing a plant-based cheese-like food product according to claim 1, wherein the acidification is lactic acid fermentation.

3. The method for producing a plant-based cheese-like food product according to claim 2, wherein the raw material containing a plant protein is derived from a legume.

4. The method for producing a plant-based cheese-like food product according to claim 2, wherein the raw material containing a plant protein is derived from a seed.

5. The method for producing a plant-based cheese-like food product according to claim 2, wherein the raw material containing a plant protein is derived from a cereal.

6. The method for producing a plant-based cheese-like food product according to claim 2, wherein an oil or fat is added as a raw material.

7. The method for producing a plant-based cheese-like food product according to claim 6, wherein a thickener is added as a raw material.

8. The method for producing a plant-based cheese-like food product according to claim 1, wherein the nuclease performs hydrolysis at a temperature of 35°C to 75°C.

## Patentansprüche

1. Verfahren zur Herstellung eines pflanzlichen käseähnlichen Lebensmittelprodukts, wobei das Verfahren umfasst Anwenden einer Nuklease auf ein Rohmaterial, das ein pflanzliches Protein enthält, wobei die Nuklease eine 5'-Phosphodiesterase ist, und dann Ansäuern des Rohmaterials auf einen pH-Wert von 3,5 bis 5,7, und wobei Rohmaterial tierischen Ursprungs weniger als 50 Gew.-% aller Rohmaterialien des pflanzlichen käseähnlichen Lebensmittelprodukts ausmacht.

2. Verfahren zur Herstellung eines pflanzlichen käseähnlichen Lebensmittelprodukts nach Anspruch 1, wobei die Ansäuerung Milchsäuregärung ist.

3. Verfahren zur Herstellung eines pflanzlichen käseähnlichen Lebensmittelprodukts nach Anspruch 2, wobei das Rohmaterial, das ein pflanzliches Protein enthält, aus einer Hülsenfrucht gewonnen wird.

4. Verfahren zur Herstellung eines pflanzlichen käseähnlichen Lebensmittelprodukts nach Anspruch 2, wobei das Rohmaterial, das ein pflanzliches Protein enthält, aus einem Samen gewonnen wird.

5. Verfahren zur Herstellung eines pflanzlichen käseähnlichen Lebensmittelprodukts nach Anspruch 2, wobei das Rohmaterial, das ein pflanzliches Protein enthält, aus einem Getreide gewonnen wird.

6. Verfahren zur Herstellung eines pflanzlichen käseähnlichen Lebensmittelprodukts nach Anspruch 2, wobei ein Öl oder Fett als ein Rohmaterial hinzugefügt wird.

7. Verfahren zur Herstellung eines pflanzlichen käseähnlichen Lebensmittelprodukts nach Anspruch 6, wobei ein Verdickungsmittel als ein Rohmaterial hinzugefügt wird.

8. Verfahren zur Herstellung eines pflanzlichen käseähnlichen Lebensmittelprodukts nach Anspruch 1, wobei die Nuklease eine hydrolytische Reaktion bei einer Temperatur von 35 °C bis 75 °C bewirkt.

## Revendications

1. Procédé de fabrication d'un produit alimentaire végétal similaire au fromage, le procédé comprenant l'application d'une nucléase à une matière première contenant une protéine végétale, dans lequel la nucléase est une 5'-phosphodiestérase, puis l'acidification de la matière première à un pH de 3,5 à 5,7, et dans lequel la matière première d'origine animale représente moins de 50 % en poids de toutes les matières premières du produit alimentaire végétal similaire au fromage.

2. Procédé de fabrication d'un produit alimentaire végétal similaire au fromage selon la revendication 1, dans lequel l'acidification est une fermentation lactique.

3. Procédé de fabrication d'un produit alimentaire végétal similaire au fromage selon la revendication 2, dans lequel la matière première contenant une protéine végétale est dérivée d'une légumineuse.

4. Procédé de fabrication d'un produit alimentaire végétal similaire au fromage selon la revendication 2, dans lequel la matière première contenant une protéine végétale est dérivée d'une graine.

5. Procédé de fabrication d'un produit alimentaire végétal similaire au fromage selon la revendication 2, dans lequel la matière première contenant une protéine végétale est dérivée d'une céréale.

6. Procédé de fabrication d'un produit alimentaire végétal similaire au fromage selon la revendication 2, dans lequel une huile ou une graisse est ajoutée en tant que matière première.

7. Procédé de fabrication d'un produit alimentaire végétal similaire au fromage selon la revendication 6, dans lequel un épaississant est ajouté en tant que matière première.

8. Procédé de fabrication d'un produit alimentaire végétal similaire au fromage selon la revendication 1, dans lequel la nucléase effectue l'hydrolyse à une température comprise entre 35 °C et 75 °C.
